(19) Europäisches Patentamt
European Patent Office
Office européen des brevets

(11) Publication number: **0 159 194**
**B1**

(12) **EUROPEAN PATENT SPECIFICATION**

(45) Date of publication of patent specification: **19.09.90**

(21) Application number: **85302706.8**

(22) Date of filing: **17.04.85**

(51) Int. Cl.[5]: **C 07 D 213/69,**
C 07 D 309/40, C 07 F 15/00,
A 61 K 31/295, A 61 K 31/35,
A 61 K 31/40

(54) **Iron 3-hydroxypyrone or 3-hydroxypyridone complexes and pharmaceutical compositions containing them.**

(30) Priority: **19.04.84 GB 8410289**

(43) Date of publication of application:
**23.10.85 Bulletin 85/43**

(45) Publication of the grant of the patent:
**19.09.90 Bulletin 90/38**

(84) Designated Contracting States:
**BE CH DE FR IT LI NL SE**

(56) References cited:
**EP-A-0 094 149**
**EP-A-0 107 458**
**EP-A-0 120 670**

**The file contains technical information submitted after the application was filed and not included in this specification**

(73) Proprietor: **NATIONAL RESEARCH DEVELOPMENT CORPORATION**
**101 Newington Causeway**
**London SE1 6BU (GB)**

(72) Inventor: **Callingham, Brian Albert**
**1 Bentley Road**
**Cambridge CB2 2AW (GB)**
Inventor: **Hider, Robert Charles**
**257 Point Clear Road St. Osyth**
**Clacton Essex (GB)**
Inventor: **Kontoghiorghes, George**
**15 Celia Road Tufnell Park**
**London N19 (GB)**
Inventor: **Stockham, Michael Arthur**
**Baltroy Stickling Green Clavering**
**Saffron Walden Essex (GB)**

(74) Representative: **Stephenson, Gerald Frederick**
**Patent Department National Research Development Corporation 101 Newington Causeway**
**London SE1 6BU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to iron compounds for use in pharmaceutical compositions for the treatment of iron deficiency anaemia.

An adequate supply of iron to the body is an essential requirement for tissue growth in both man and animals. Although there is normally an ample amount of iron in the diet, the level of absorption of iron from food is generally low so that the supply of iron to the body can easily become critical under a variety of conditions. Iron deficiency anaemia is commonly encountered in pregnancy and may also present a problem in the newly born, particularly in certain animal species such as the pig. Moreover, in certain pathological conditions there is a mal distribution of body iron leading to a state of chronic anaemia. This is seen in chronic diseases such as rheumatoid arthritis, certain haemolytic diseases and cancer.

Although a wide range of iron compounds is already marketed for the treatment of iron deficiency anaemia, the level of iron uptake by the body from these compounds is often quite low, necessitating the administration of relatively high dosage levels of the compound. The administration of high dose, poorly absorbed, iron complexes may cause siderosis of the gut wall and a variety of side effects such as nausea, vomiting, constipation and heavy malodorous stools.

In the European patent applications of numbers EP—A—94 149, EP—A—107 458 and EP—A—120 670, we described iron complexes of various 3-hydroxpyrid-2-ones, 3-hydroxyprid-4-ones and 3-hydroxy-4-pyrones which we have identified as being of particular value for use as relatively low dosage levels in the treatment of iron deficiency anameia. It has now been found that certain advantages, as discussed hereinafter, accrue from the use of complexes not described in these earlier applications which contain one or more hydroxypyridone or hydroxypyrone ligands but in which the ligands present in the complex are not identical.

According to the present invention a neutral 3:1 ligand:iron(III) complex comprises three monobasic bidentate ligands, of which at least two are different, each ligand separately being provided by a compound which is:

(1) a 3-hydroxy-4-pyrone of formula (I)

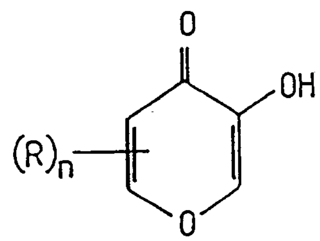

(I)

in which each R separately is an aliphatic or cycloaliphatic hydrocarbon group of 1 to 6 carbon atoms and n is 0, 1, 2 or 3;

(2) a 3-hydroxypyridone of formula (II) or (III)

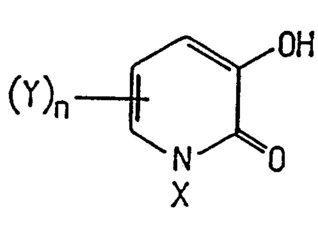

(II)

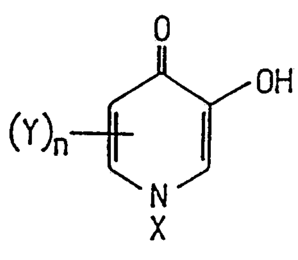

(III)

in which each Y separately is a $C_{1-6}$ aliphatic or cycloaliphatic hydrocarbon group or a $C_{1-3}$ alkyl group substituted by a $C_{1-4}$ alkoxy, halogen or hydroxy group.

X is a $C_{1-6}$ aliphatic or cycloaliphatic hydrocarbon group, a formyl or ($C_{1-4}$ alkyl)-carbonyl group, or a $C_{1-8}$ aliphatic or cycloaliphatic hydrocarbon group substituted by one or more substituents selected from formyl, ($C_{1-4}$)-carbonyl, $C_{1-4}$ alkoxy, carbamoyl, sulphamoyl, mono- and di- $C_{1-6}$ aliphatic or cycloaliphatic hydrocarbyl N-substituted carbamoyl and N-substituted sulphamoyl, formylamino, ($C_{1-6}$ aliphatic or cycloaliphatic hydrocarbyl)-carbonylamino, ($C_{1-6}$ aliphatic or cycloaliphatic hydrocarbyl)-sulphonylamino, mono-$C_{1-6}$ aliphatic or cycloaliphatic hydrocarbyl N-substituted formylamino, N-substituted ($C_{1-6}$ aliphatic or cycloaliphatic hydrocarbyl)-carbonylamino and N-substituted ($C_{1-6}$ aliphatic or cycloaliphatic hydrocarbyl)-sulphonylamino, formyloxy, ($C_{1-6}$ aliphatic or cycloaliphatic hydrocarbyl)-carbonyloxy, ($C_{1-6}$ ali-

phatic or cycloaliphatic hydrocarbyl)oxycarbonyl, ($C_{1-6}$ aliphatic or cycloaliphatic hydrocarbyl)-sulphonyloxy, ($C_{1-6}$ aliphatic or cycloaliphatic hydrocarbyl)-oxysulphonyl, halogen and hydroxy groups, and n is 0, 1, 2 or 3, but excluding compounds in which the grouping

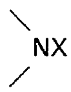 contains either a group 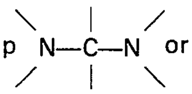 or a group 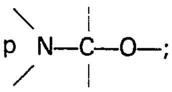; or

(3) lactic acid, gluconic acid, ascorbic acid, glycine, leucine, isoluecine, methionine, phenylalanine, tyrosine, valine or a di- or tri-peptide in which the amino acid residues, which may be the same or different, are residues of amino acids selected from this last mentioned group of seven amino acids; but with the proviso that at least one of the ligands is of type (1) or (2).

It should be appreciated that a feature of the iron complexes of the present invention is that they are neutral, i.e. there is an internal balance of charges between the ferric cation and the ligands bound covalently thereto, there being no need for any additional non-covalently bound anion or anions, such as chloride, to balance the charge on the ferric cation. The hydroxypyridone and hydroxypyrone ligands are each bidentate and monobasic (the ligand containing a group $—O^-$ in place of the group —OH present in the compound itself).

It will be appreciated that the 3:1 ligand:iron complexes of the present invention may contain various different combinations of the ligands of the types (1) to (3) described above, subject to the requirement that at least one is hydroxypyridone or hydroxypyrone ligand and that at least two of the three ligands are not identical. Thus, all of the ligands may be of type (1) or of type (2), although differing within these types, or the ligands may be of all three types. Alternatively, the ligands may be a mixture of types (1) and (2), a mixture of types (1) and (3) or a mixture of types (2) and (3). It will most usually be the case that two of the monobasic, bidentate ligands present in the 3:1 complex will be the same, with the third one being different. Moreover, at least two will preferably be of the hydroxypyridone or hydroxypyrone type. Most conveniently, therefore, the complex contains three ligands of type (2) or particularly of type (1) which are derived from two different compounds, or the complex contains two identical ligands of type (2) or particularly of type (1) together with a third ligand which is either of type (1) or (2), respectively, or less preferably of type (3).

The ability of both the metal-free ligand-providing compounds and of the iron complex to permeate membranes is important in the context of the treatment of iron deficiency anaemia and it is also desirable for both to possess some degree of water solubility. A good indication of the physical properties of a ligand-providing compound and of an iron complex in this respect is provided by the value of the partition coefficient ($K_{part}$) obtained on partition between n-octanol and tris hydrochloride (20 mM, pH 7.4; tris representing 2-amino-2 hydroxymethylpropane 1,3-diol) at 20°C and expressed as the ratio (concentration in organic phase)/(concentration in aqueous phase). Preferred complexes show a value of $K_{part}$ for each ligand-providing compound of above 0.02 or 0.05 but less than 3.0, especially of above 0.2 but less than 1.0, together with a value of $K_{part}$ for the 3:1 iron(III) complex of about 0.02 but less than 6.0, especially of above 0.1 or 0.2 but less than 1.0. For examples of measured partition coefficients of metal-free hydroxypyridones and hydroxypyrones, and of iron complexes thereof in which all of the ligands are identical, reference should be made to Example 1 of each of the three applications referred to hereinbefore. The following comments upon preferences among the different ligands of types (1) to (3) which may be used in complexes according to the present invention are made in the light of these preference as to partition coefficients.

The hydroxypyrone ligands of type (1) are of particular value and the complexes according to the present invention may conveniently contain at least one such ligand. The substituted 3-hydroxy-4-pyrones may carry more than one type of aliphatic or cycloapliphatic hydrocarbon group but this is not usual and, indeed, substitution by two rather than three, and particularly by only one aliphatic or cycloaliphatic hydrocarbon group is preferred. The term aliphatic or cycloaliphatic hydrocarbon group is used herein to include both acyclic and cyclic groups which may be unsaturated or saturated, the acyclic groups having a branched chain or especially a straight chain. Groups of from 1 to 4 carbon atoms and particularly of 1 to 3 carbon atoms are of most interest. Saturated aliphatic or cycloaliphatic hydrocarbon groups are preferred, these being either cycloalkyl groups (which term is used herein to denote a saturated cycloaliphatic hydrocarbon group) such as cyclopropyl and especially cyclohexyl or, more particularly, alkyl groups (which term is used herein to denote a saturated aliphatic hydrocarbon group) such as n-propyl and isopropyl, and especially ethyl and methyl. Substitution at the 2- or 6-position is of especial interest although, when the ring is substituted by the larger aliphatic or cycloaliphatic hydrocarbon groups, there may be an advantage in avoiding substitution on a carbon atom alpha to the

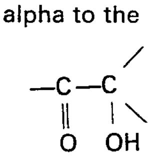

system.

This system is involved in the complexing with iron and the close proximity of one of the larger aliphatic hydocarbon groups may lead to steric effcets which inhibit complex formation.

Preferred hydroxypyrones providinig ligands present in complexes according to the present invention have the formula (I), specific hydroxypyrones of particular interest having the formulae (IA) and (IB)—

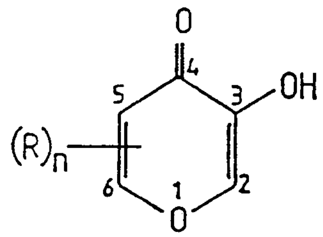
(I)

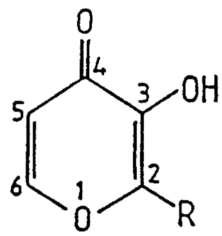
(IA)

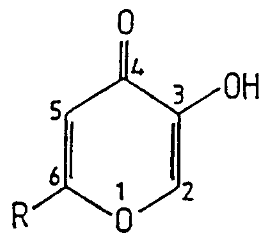
(IB)

in which
R is a $C_{1-6}$ alkyl group, for example methyl, ethyl, n-propyl isopropyl or butyl, and n is 0, 1, 2 or 3 (the ring being unsubstituted by any alkyl group when n is 0). Among these compounds 3-hydroxy-2-methyl-4-pyrone (maltol; IA, R = $CH_3$) is of most interest, whilst 3-hydroxy-4-pyrone (pyromeconic acid; I, n =0) 3-hydroxy-6-methyl-4-pyrone (IB, R = $CH_3$) and particularly 2-ethyl-3-hydroxy-4-pyrone (ethylpyromeconic acid; IA, R = $C_2H_5$) are also of especial interest.

As regards the hydroxypyridone ligands of type (2), these correspond to the hydroxypyridone ligands described in GB 2136806A and in its exact equivalent EP—A—120670 (see also GB 2117766A and its exact equivalent EP—A—120670). GB 2117766A refers to 3-hydroxypyrid-2-ones and 3-hydroxypyrid-4-ones in which the hydrogen atom attached to the nitrogen atom is replaced by an aliphatic hydrocarbon group of 1 to 6 carbon atoms and, optionally, in which one or more of the hydrogen atoms attached to ring carbon atoms are also replaced by the same or a different aliphatic hydrocarbon group of 1 to 6 carbon atoms, whilst GB 2136806A refers to 3-hydroxypyrid-2-ones and 3-hydroxypyrid-4-ones which are substituted as defined under (2) hereinbefore but excluding those compounds in which the replacement of hydrogen atoms is effected only by aliphatic hydrocarbon groups (these compounds being the substituted hydroxypyridones of GB 2117766A).

Hydroxypyridones providing ligands which may be used in complexes according to the present invention have the formula (II) and (III):—

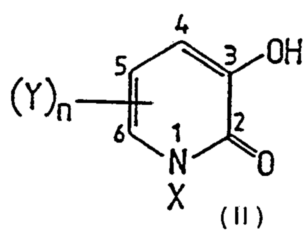
(II)

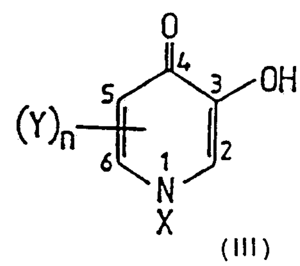
(III)

in which X and Y are substituents as defined hereinbefore in relation to possible C- and N-substituents or 3-hydroxypyridones and n is 0, 1, 2 or 3, the hydroxypyrid-2-ones generally being of somewhat greater interest than the 3-hydroxypyrid-4-ones.

Preferences as to the nature and position of the substituent groups present in the hydroxypyridones are broadly as expressed in the two earlier applications. Thus, substituted aliphatic or cycloaliphatic hydrocarbon groups present in the hydroxypyridones may as indicated carry more than one subsitutent group but it is preferred that two rather than three, and particularly only one substituent group is present. Such substituted aliphatic or cycloaliphatic hydrocarbon group substituents contain groups of 1 to 8 and particularly of 1 to 6 carbon atoms, but the simpler hydroxypyridones of GB 2117766A and EP—A—94149 containing only unsubstituted aliphatic or cycloaliphatic hydrocarbon group substituents are of the greatest interest. The preference among the aliphatic or cycloaliphatic hydrocarbon groups present in these hydroxypyridones correspond largely to those expressed in relation to the hydroxypyrones, with methyl groups conveniently being used for substitution on ring carbon atoms but larger alkyl groups also being of particular interest for substitution on the ring nitrogen atoms. Substitution of the ring carbon atoms, which is again preferably by one rather than two or three aliphatic hydrocarbon groups, is of particular interest in the case of the 3-hydroxypyrid-4-ones, for example at the 6- or particularly the 2-position, whilst the 3-hydroxypyrid-2-ones may more often be used without any additional aliphatic or cycloaliphatic hydrocarbon group substituent on the ring carbon atoms.

Specific hydroxypyridones of particular interest have formulae (IIA), (IIIA) and (IIIB):—

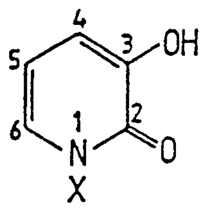
(IIA)

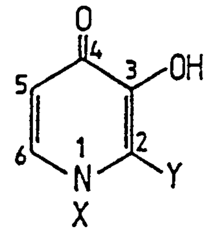
(IIIA)

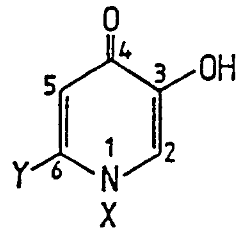
(IIIB)

in which X is a $C_{1-6}$ alkyl group, for example methyl, ethyl, n-propyl, isopropyl or butyl, and Y is hydrogen or particularly a $C_{1-6}$ alkyl group, for example methyl. Among such compounds 1-ethyl-3-hydroxy-pyrid-2-one, 3-hydroxy-1-propylpyrid-2-one, 3-hydroxy-1-(1′-methylethyl)-pyrid-2-one, 1-butyl-3-hydroxypyrid-2-one, 1-ethyl-3-hydroxy-2-methylpyrid-4-one, 3-hydroxy-2-methyl-1-propylpyrid-4-one, 3-hydroxy-1-(1′-methylethyl)-2-methylpyrid-4-one and 1-butyl-3-hydroxy-2-methylpyrid-4-one are of particular interest with the 3-hydroxypyrid-2-ones such as 1-ethyl-3-hydroxypyrid-2-one being especially preferred.

The ligands of type (3) may be derived from various forms of compound, many of which are naturally occurring, and include the hydroxy acids, lactic acid and gluconic acid, and the amino acids, glycine, isoleucine, leucine, methionine, phenylalanine, tyrosine and valine. Also of use in providing ligands of type (3) are certain peptides, in particular tri- and especially di-peptides, containing the same or different amino acids selected from those listed above such as glycyl-leucine, leucyl-glycine and especially glycyl-glycine and leucyl-leucine. A further compound of particular interest for providing a type (3) ligand is ascorbic acid (vitamin C). It should be noted that ascorbic acid is capable of providing a dibasic rather than a monobasic anion but compounds such as this are quite suitable for use in providing ligands of type (3) where they have a single pKa, only, which is less than 10 since, in use under physiological conditions, the ascorbate or other such anion will be monobasic. In selecting compounds for providing type (3) ligands, the more hydrophobic compounds are generally of greater interest so that among the amino acids, for example, the more complex amino acids than glycine may be of greater value.

Examples of specific iron complexes according to the present invention are (1-ethyl-3-hydroxypyrid-2-one) (1-butyl-3-hydroxy-pyrid-4-one) iron(III), $(maltol)_2$ (1-ethyl-3-hydroxypyrid-2-one) iron (III), $(maltol)_2$ (leucine) iron(III), $maltol)_2$ (glycine) iron(III), $(maltol)_2$ (ascorbic acid) iron(III), $(maltol)_2$ (gluconic acid) iron (III) and especially $(maltol)_2$ (ethylpyromeconic acid) iron(III) and (ethylpyromeconic $acid)_2$ maltol iron(III). It will be appreciated that "maltol" is used in the names of these complexes to represent the ligand derived from maltol, and similarly for the the ligands, this usage being employed throughout the specification.

The iron complexes are conveniently prepared by the reaction of a mixture of the compounds which provide the ligands and iron ions, the latter conveniently being derived from an iron salt, particularly a ferric halide and especially ferric chloride. The reaction is conveniently effected in a suitable mutual solvent and water may be used for this purpose. If desired, however, an aqueous/organic solvent mixture may be used or an organic solvent, for example ethanol, methanol or chloroform and mixtures of these solvents together and/or with water where appropriate. In particular, methanol or especially ethanol may be used where it is desired to effect the separation of at least a major part of a by-product such as sodium chloride by precipitation whilst the iron complex is retained in solution.

The nature of the product obtained will depend in part upon the molar proportion of the various reactants but also upon the pH of the reaction medium. Thus, to prepare the ferric complexes containing a 3:1 molar proportion of ligand:iron(III), the ligand-providing compounds and the ferric salt are conveniently mixed in solution in a 3:1 molar proportion and the pH adjusted to a value in the range of 6 to 9, for example 7 or 8. If a similar excess of the compounds:iron is employed but no adjustment is made of the acidic pH which results on the admixture of the compounds and an iron salt such as ferric chloride, then a mixture of 2:1 and 1:1 complexes will instead by obtained. Adjustment of the pH may conveniently be effected by the addition of sodium carbonate as described hereinafter in Example 1. However, a possible alternative, which is of particular interest when preparing the iron complexes in batches of 20 g or more, is to use a hydroxide base such as sodium or ammonium hydroxide. When using a hydroxide base, the reaction may conveniently be carried out in 4:1 v/v ethanol:water as a solvent and the pH adjusted by the addition of a 2 molar aqueous solution of the base. It will be appreicated that the presence of a proportion of water in the reaction mixture will lead to retention of a by-product in the iron complex on evaporation of the solvent (a chloride where the iron salt is ferric chloride). However, this can be removed, if desired, by procedures such as crystallisation from a suitable solvent system or sublimation in the particular case of ammonium chloride.

The individual ligand providing compounds may conveniently be used in a 1:1:1 or 2:1 molar proportion, with a 1 molar proportion of the ferric salt, depending on whether all three of the ligands are different or, as is usually preferred, two of the ligands are the same and the third is different. It will be appreciated, however, that the use of such proportions will not lead exclusively to the 1:1:1 or a single 2:1

complex since, although these forms of complex will predominate providing the ligand-providing compounds are of similar reactivity, they will be obtained in admixture with other forms of complex as discussed hereinafter. Indeed, if it is desired to enhance the degree of admixture of different forms of complex which is obtained, the proportions of reactants may be varied to this end. Thus, for example, a 1.5:1.5 molar proportion of two different ligands may be used to encourage the formation of a mixture of the two possible types of 2:1 complex differing in the ligand which predominates.

Reaction to form the iron complex is generally rapid and will usually have proceeded substantially to completion after 5 minutes at about 20°C, although a longer reaction time may be used if necessary. Following separation of any precipitated by-product, such as sodium chloride in the case of certain solvent systems, the reaction mixture may conveniently be evaporated on a rotary evaporator to yield the iron complex which will usually be an oil initially which will, however, often form a glass on standing. The present invention thus further includes a process for the preparation of an iron complex as described hereinbefore, which comprises reacting a mixture of selected hydroxypyridone, hydroxypyrone and alternative ligand-providing compounds, as described hereinbefore, with ferric ions and isolating the resultant complex.

Whilst for some uses it may be appropriate to prepare the iron complex in a form free from by-products of manufacture apart from other complexes, in other cases, for example with a solid oral formulation as described hereinafter, the presence of by-products such as sodium chloride may be quite acceptable. In general, however, the neutral 3:1 iron(III) complex is of particular interest in a form which is substantially free at least from those by-products which are complexes containing different overall proportions of ligand:iron. Thus the 3:1 complex, although usually obtained and used in a form in which it is in admixture with other types of 3:1 complex are discussed hereinafter, is preferably substantially free from 2:1 and 1:1 complexes. In addition, the isolation of the complex will usually provide it in a form substantially free from any metal-free compound corresponding to a ligand present in the complex. The term "substantially free from" is used herein to indicate the presence of 10% by weight or less of the material referred to.

Certain of the ligand-providing compounds, such as maltol, are available commercially. With others, routes for their preparation are described in the three UK patent applications referred to hereinbefore. Thus, for example, with the hydroxypyrones a convenient starting material in many instances consists of pyromeconic acid which is readily obtainable by the decarboxylation of meconic acid and may be reacted with an aldehyde to insert a 1-hydroxyalkyl group at the 2-position, which group may then be reduced to produce a 2-alkyl-3-hydroxy-4-pyrone. The preparation of 2-ethyl-3-hydroxy-4-pyrone, etc, by this route is described in US application serial number 310,141 (series of 1960).

It will be appreciated that these are not the only routes available to these compounds and their iron complexes and that various alternatives may be used as will be apparent to those skilled in the art. Moreover, it will be appreciated that certain of the compounds may be converted *in vivo* to other compounds which are responsible for the metal binding activity observed *in vivo*. This will be true, for example, of compounds containing ester groups which are likely to be converted to carboxy groups when the compounds are administered orally.

The iron complexes of the present invention are of particular interest for several reasons. Firstly, the inclusion in a complex of a mixture of different ligands provides an added dimension to the design of complexes having optimised properties for take up *in vivo* to provide a controlled supply of iron applicable in a particular human or veterinary context. More specifically, comparative results obtained in human erthrocytes and in non-everted rat jejunal segments suggest that complexes containing mixed ligands may provide a more available source of iron than iron complexes in which the ligands are homogeneous. Secondly, apart from the behaviour of the complexes *in vivo*, the present invention provides particular advantages in relation to the formulation of iron complexes. In certain contexts, as discussed in more detail hereinafter, liquid formulations of the iron complexes are of particular interest, for example for oral veterinary administration and particularly for parenteral verterinary and human administration, and it has been found that for use in such contexts the solubility of some of the iron complexes of the three previously mentioned UK patent applications is less than might have been desired.

The mixed ligand complexes of the present invention generally show much higher solubilities, both in water and in organic solevents, as compared with those in which the ligands are homogeneous. It is believed that the reason for this lies in the diversity of different stereoisomers of one complex which can arise when a mixture of ligands is present and which can be augmented by the presence of several different complexes in a reaction mixture obtained from the reaction with iron ions of more than one ligand producing compound. Thus, a 3:1 iron(III) complex containing three identical asymmetric ligands can exist in four stereoisomeric forms but when a complex is produced by the reaction of a 3 molar proportion of a mixture of two ligand-producing compounds (A and B) with ferric ions then the following types of complex may be present in the reaction mixture: $FeA_3$, $FeB_3$ and $FeB_2A$. Moreover, although the first two mentioned complexes will exist in four stereoisomeric forms, the last two mentioned complexes will each exist in eight stereoisomeric forms (an even more complex mixture will result if three different ligands are present). It has been found that the four stereoisomers of a $FeA_3$ or $FeB_3$ complex will co-crystallise with ease but that for a $FeAB_2$ or $FeB_2A$ complex the increased number of stereoisomers, and the presence of other 3:1 ligand:iron (III) complexes, prevents such co-crystallisation and ensures that the product is a liquid with enhanced solubility as compared with the usually solid $FeA_3$ and $FeB_3$ complexes.

The iron complexes according to the present invention may be formulated for use as pharmaceuticals for both veterinary, for example in an avian or particularly a mammalian context, and human use by a variety of methods. For instance, they may be applied as an aqueous, oily or emulsified composition incorporating a liquid diluent which may often be employed for parenteral administration and therefore may conveniently be sterile and pyrogen free. Oral administration is often preferred for the treatment of iron deficiency anaemia in humans and the complexes of the present invention may be given by such a route. Although compositions incorporating a liquid diluent may be used for oral administration, it is more usual, at least in humans, to use compositions incorporating a solid carrier, for example a conventional solid carrier material such as starch, lactose, dextrin or magnesium stearate. Such solid compositions may conveniently be of a formed type, for example as tablets, capsules (including spansules), etc.

Although solid compositions may be preferred for the treatment of iron deficiency anaemia in certain contexts, liquid compositions are of interest in other contexts, for example in human and veterinary intramuscular administration and in veterinary oral administration as discussed hereinafter. It is in the area of liquid compositions that the present invention is of particular, although certainly not exclusive, interest. Thus, it is often desirable to produce liquid compositions containing a higher concentration than is readily obtainable with a purely aqueous composition or indeed one containing organic solvents such as simple monohydric alcohols. It has been found with the iron complexes described in the three UK patent applications mentioned hereinbefore that higher concentrations may be achieved by the use of solvents containing two or more hydroxy groups or a hydroxy and an ether group, especially glycols or glycol ethers, either in admixture with water or, for better solubilisation, alone. The glycol ethers of particular interest are the mono-ethers containing as an etherifying group an aliphatic hydrocarbon group of 1 to 6 carbon atoms as described above, for example a methyl group, such as glycol mono-ether being methyl ethylene glycol. In general, however, the glycols themselves are preferred. Examples of such glycols are the simple dihydroxy alkanes such as ethylene glycol as well as those more complex compounds comprising two hydroxy groups attached to a chain containing both carbon and oxygen atoms, such as triethylene glycol, tetraethylene glycol and polyethylene glycol, for example of 4000 daltons molecular weight. Triethylene glycol and especially tetraethylene glycol are of particular interest in view of their very low toxicity. By using such glycols and glycol ethers it is possible to increase solubility for many complexes to 10 to 20 mg/ml. Although such techniques may also be employed in the formulation of the iron complexes according to the present invention their greater solubility will often allow one with advantage to employ simpler forms of liquid composition and still achieve concentrations considerably in excess of this 10 to 20 mg/ml range.

As indicated, liquid compositions are of particular interest in relation to parenteral administration, a requirement for which arises with humans in certain contexts but also particularly in a veterinary context, for example with pigs. The problems of iron deficiency anaemia in newly born pigs arise primarily during the first three weeks or so of their life when a very rapid weight gain takes place. The usual routes for administration of the iron complexes of the present invention to young piglets are parenteral, for example intramuscular, or oral, for example as a liquid preparation "injected" into the mouth. However, an alternative approach is to enhance the iron content of the milk on which the piglets are feeding by treating the mother pig using oral or parenteral administration, for example, with an injectable slow release preparation (such an approach may also be of interest in a human context). When it is applicable to feed piglets on foodstuffs other than the milk of the mother pig, it may also be possible to effect the pharmaceutical administration of the iron complex in this other foodstuff.

Other forms of administration than by injection or through the orgal route may also be considered in both human and veterinary contexts, for example the use of suppositories or pessaries for human administration. Another form of pharmaceutical composition of some particular interest is one for buccal or nasal administration and such compositions are discussed hereinafter in more detail.

Compositions may be formulated in unit dosage form, i.e. in the form of discrete portions containing a unit dose, or a multiple or sub-unit of a unit dose. Whilst the dosage of the iron complex given will depend on various factors, including the particular compound which is employed in the composition, it may be stated by way of guidance that maintenance of the amount of iron present in the human body at a satisfactory level will often be achieved using a daily dosage, in terms of the iron content of the compound, which lies in a range from about 0.1 to 10 mg and often in a range of from 0.5 to 10 mg, for example 1 or 2 mg, veterinary doses being on a similar g/Kg body weight ratio. However, it will be appreciated that it may be appropriate under certain circumstances to give daily dosages either below or above these levels. In general, the aim should be to provide the amount of iron required by the patient without administering any undue excess and the properties of the pharmaceutical compositions according to the present invention are particularly suited to the achievement of this aim.

It will be appreciated from the foregoing discussion that more than one iron complex according to the present invention may be contained in the pharmaceutical composition, or indeed, other active compounds may be included in the composition, for example compounds having the ability to facilitate the treatment of anameia, such as folic acid. Another additional component which may be included in the composition, if desired, is a source of zinc. Iron compounds used in the treatment of iron deficiency anameia can inhibit the mechanism of zinc uptake in the body and this can cause serious side effects in the foetus when treating anaemia in a pregnant female. It is believed, however, that the iron complexes of the present invention

have a further advantage in that they either do not have this effect or exhibit the effect at a lower level than the compounds at present used in the treatment of anaemia. Accordingly, it may often be the case that the level of zinc-providing compound added to the composition may not require to be high or, with preferred formulations of the iron complexes, may be dispensed with altogether.

It has never before been appreciated that the novel iron complexes described herein might be used, and with great advantage, in a pharamaceutical context. Accordingly the present invention includes an iron complex as defined hereinbefore for use in medicine, particularly in the treatment of iron anaemia deficiency.

The iron complexes described herein are particularly suited to the treatment of iron anaemia deficiency both in humans and also in a veterinary context, particularly for the treatment of various mammalian species and especially pigs. The complexes will partition into n-octanol indicating that they are able to permeate biological membranes, this property may be confirmed in practice by tests of the ability of the $^{59}Fe$ labelled iron complexes to permeat erthrocytes. The ability of the compounds in this respect will depend on the nature of the ligands present therein and the reflection of this ability in the $K_{part}$ values of various compounds has been reflected to hereinbefore.

Certain aspects of their formulation may enhance the activity of the iron complexes in particular contexts. Thus, although the neutral ferric complexes containing a 3:1 molar proportion of ligand:iron(III) are of particular value as being stable over a wide pH range from about 4 to 5 up to 10, if administered orally they will dissociate at the pH values of less than 4 prevailing in the stomach to form a mixture of the 2:1 and 1:1 complex together with free ligand. Firstly, one of several variations may be employed which avoid or reduce exposure of the iron complex to the acidic conditions of the stomach. Such approaches may involve various types of controlled release system, ranging from one, which may for example be based on a polymer, which simply provides a delayed release of the complex with time, through a system which is resistant to dissociation under acidic conditions, for example by the use of buffering, to a system which is biased towards release under conditions such as prevail in the small intestine, for example a pH sensitive system which is stabilised towards a pH of 1 to 3 such as prevails in the stomach but not one of 7 to 9 such as prevails in the small intestine. Since the pH of the stomach is higher after a meal, it may be advantageous, whatever method of formulation is used, to administer the iron complexes at such a time.

A particularly convenient approach to a controlled release composition involves encapsulating the iron complex by a material which is resistant to dissociation in the stomach but which is adapted towards dissociation in the small instestine (or possibly, if the dissociation is slow, in the large intestine). Such encapsulation may be achieved with liposomes, phospholipids generally being resistant to dissociation under acidic conditions. The liposomally entrapped 3:1 iron(III) complexes can therefore survive the acid environment of the stomach without dissociating to the 2:1 and 1:1 complexes, and the free hydroxypyrone. On entry into the small intestine the pancreatic enzymes rapidly destroy the phospholipid-dependent structure of the liposomes thereby releasing the 3:1 complex. Liposome disruption is further facilitated by the presence of bile salts. However, it is usually more convenient to effect the encapulsation, including microencapsulation, by the use of a solid composition of a pH sensitive nature.

The preparation of solid compositions adapted to resist dissociation under acidic conditions but adapted towards dissociation under non-acidic conditions is well known in the art and most often involves the use of enteric coating, whereby tablets, capsules, etc or the individual particles or granules contained therein, are coated with a suitable material. Such procedures are described, for example, in the article entitled "Production of enteric coated capsules" by Jones in Manufacturing Chemist and Aerosol News, May 1970, and in such standard reference books as "Pharmaceutical Dosage Forms", Volume III by Liebermann and Lackmann (published by Marcel Decker). One particular method of encapsulation involves the use of gelatine capsules coated with a cellulose acetate phthalate/diethylphthalate layer. This coating protects the gelatin capsule from the action of water under the acid conditions of the stomach where the coating is protonated and therefore stable. The coating is however destabilised under the neutral/akaline conditions of the intestine where it is not protonated, thereby allowing water to act on the gelatin. Once released in the intestine the rate of permeation of the intestine wall by the water soluble 3:1 iron(III) complex is relatively constant irrespective of the position within the intestine, i.e. whether in the jejunum, ileum or large instestine. Other examples of methods of formulation which may be used include the use of polymeric hydrogel formulations which do not actually encapsulate the iron complex but which are resistant to dissociation under acidic conditions.

A second approach to countering the effect of the acidic conditions prevailing in the stomach is to formulate the iron complexes as a pharmaceutical composition adapted for buccal or nasal administration. Thus, since the buccal and nasal cavities represent an environment with a pH in the region of 7, a 3:1 neutral iron(III) complex will be taken up by the membranes of the buccal cavity (including the tongue) and the nasal passages in the neutral form, without any significant degree of disproportionation to the corresponding 2:1 and 1:1 complexes. Such a form of administration thus often provides a simple approach to the protection of the 3:1 neutral iron(III) complexes from the strongly acid environment of the stomach.

A further advantage of compositions for buccal or nasal administration is that they provide some form of safety measure as regards overdoses, for example those arising from the taking by children of mediciation prescribed for adults in the same household. This can pose a considerable problem with

existing iron preparations and, although iron complexes in any case generally have the advantage of lower toxicity and lower unit dosage levels, the compositions of the present invention provide an added advantage. Thus, it is difficult rapidly to ingest a large quantity of the iron complex from the buccal cavity or nasal passages and overdosage problems are more likely to arise through swallowing the medicament. If this is done, however, and the iron complex is not formulated in such a way as to protect it from the acid environment of the stomach, disproportionation to the 2:1 and 1:1 complexes will occur thereby significantly reducing the level of iron uptake from the overdose. The generally lower toxicity of the iron complexes will avoid much of the local damage to the gastrointestinal tract which can occur in such circumstances with many commercial iron preparations.

Diluents and carriers suitable for the formulation of compositions for buccal or nasal administration may include various materials in current use in compositions for such administration. Buccal administration is of particular interest and in this case a solid composition is preferred. Such compositions adapted for retention in the mouth rather than swallowing, and consequent release of the active component in the buccal cavity, may take very many forms. These include chewing or bubble gum, lollipops, boiled sweets, effervescent tablets and particularly pastilles and lozenges. Most usually, therefore, the composition will be chewed or sucked to lead to release of the iron complex in the mouth, although it is possible to use tablets, for example in the form of a disc of polymeric material, which are attached to the inside of the buccal cavity and which gradually release the iron complex without being sucked. If desired, liquid compositions may be used in the buccal cavity, particularly aerosol sprays, but these are of less interest. All of these forms of compositions are taken through the mouth but, in contrast to the oral compositions described earlier, are adapted to release of the iron complex in the mouth rather than on being swallowed (although in the process of chewing, sucking etc a proportion of the iron complex may of course pass through the stomach). Preferred forms of compositions are pastilles and lozenges and such compositions are sometimes described by the term "linguet", this being a composition suitable for sublingual use.

Specific carriers which may be used in pastilles and lozenges are described in various tests including the British Pharmacoepia, the British Pharmacoepia Codex and Martindale, the Extra Pharmacopoeia. One particular example of a base for pastilles is described in 1980 British Pharmacoepia and consists of a mixture of gelatin, glycerine, sugar, citric acid and amaranth. The rate at which the pastille dissolves in the mouth may be varied as desired with a view to achieving a good level of uptake of iron, the rate of dissolution being reduced, for example, by increasing the proportion of gelatin used. Pastilles may conveniently be prepared by forming a melt containing a suitable amount of iron complex and the carrier and then pouring this into a mould and allowing to dry. One particular example of a base for lozenges is described in the 1959 British Pharmacoepia Codex and consists of a mixture of sucrose, acacia and rose oil water. Once again, the rate of dissolution may be controlled by variation of the ingredients in the base material. Lozenges may conveniently be prepared either by forming a "dough" from which the lozenges are cut or, preferably, by compression. If desired, further flavourings can be incorporated in the pastilles or lozenges but the taste of the iron complexes is so acceptable that this may be unnecessary, except perhaps for paediatric formulations.

Where compositions for nasal administration are employed these will usually be liquid and may comprise water and/or suitable organic solvents. Such compositions may conveniently be used either as drops or in the form of an aerosol spray. It is, however, possible to use solid compositions in the form of a snuff if so desired.

A third approach to countering the effect of the acidic conditions prevailing in the stomach, which is described in EP—A—94149 and which may also have certain other advantages described therein, is to formulate the iron complex in the pharmaceutical composition together with one or more of the metal-free ligand-providing compounds from which it is derived. Although, this approach may also be contained with the iron complexes of the present invention, particularly when using a metal-free compound of type (1) or (2), it is perhaps rather less attractive in this case in view of the possibility of effecting an exchange between the metal-free compound and the bound ligands, particularly those of type (3), to produce a different type of complex. Also described in EP—A—94149 is the use of an iron complex in admixture with a different metal-free iron chelating agent. Once again, this approach may also be considered with the iron complexes of the present invention but is less attractive for similar reasons.

In addition to the pharmaceutical uses of the iron complexes discussed above they are also of potential interest as a source of iron in various other contexts including in cell and bacterial growth, in plant growth, as a colouring agent and in the control of iron transport across membranes.

This invention is illustrated by the following Examples.

## Examples

### Example 1

Preparation of the Iron Complexes

(A) An ethanolic solution of ferric chloride is reacted for 5 minutes at room temperature with a chloroform solution containing 2 molar equivalents of maltol (3-hydroxy-2-methyl-4-pyrone) and 1 molar equivalent of 1-ethyl-3-hydroxylpyrid-2-one[(1)]. The resultant solution is neutralised by the addition of solid

sodium carbonate with stirring, the precipitated sodium chloride is removed by filtration and the filtrate is evaporated to give, as an oil, an essentially quantitative yield of a mixture of 3:1 complexes in which the $(maltol)_2$ (1-ethyl-3-hydroxypyrid-2-one) iron(III) complex predominates. This oil solidifies on standing to give a glass, $v_{max}$(glass) 1525, 1560, 1620, 1645 $cm^{-1}$. (The infra-red spectrum of this compound, and of the other compounds described hereinafter, is obtained by dissolving the original glass in chloroform and evaporating this solution *in situ* on the sodium chloride plates to re-form a glass on which the infra-red spectrum is run).

The neutral ferric complexes of (a) ethylpyromeconic acid (2-ethyl-3-hydroxy-4-pyrone) and maltol and (b) 1-(ethyl-3-hydroxy-pyrid-2-one and 1-butyl-3-hydroxypyrid-4-one are similarly prepared by procedure (A) to give (a) a mixture of 3:1 complexes in which the (ethylpyromeconic acid)$_2$ (maltol) iron(III) complex predominates, as a glass, and (b) a mixture of 3:1 complexes in which the (1-ethyl-3-hydroxypyrid-2-one)$_2$ (1-butyl-3-hydroxypyrid-4-one) iron(III) complex predominates, as a glass, $\beta_{max}$ 1525, 1530, 1610 $cm^{-1}$.

(B) An ethanolic solution of ferric chloride is treated with a hot (60°C) ethanolic solution containing 2 molar equivalents of maltol and 1 molar equivalent of ascorbic acid[1] (the use of hot ethanol, rather than old chloroform as in procedure (A), is required to dissolve the acorbic acid) and the mixture allowed to cool to room temperature over 30 minutes. The resultant cold solution is neutralised by the addition of solid sodium carbonate with stirring, the precipitated sodium chloride is removed by filtration and the filtrate is evaporated to give, as an oil, an essentially quantitative yield of a mixture of 3:1 complexes in which the $(maltol)_2$ (ascorbic acid) iron(III) complex predominates. This oil solidifies on standing to give a glass, $v_{max}$(glass) 1500, 1560, 1600, 1790 $cm^{-1}$.

The neutral ferric complexes of (a) a maltol and leucine and (b) maltol and glycine are similarly prepared by procedure (B) to give (a) a mixture of 3:1 complexes in which the $(maltol)_2$ (leucine) complex predominates, as a glass, $v_{max}$ 1500, 1560, 1595 $cm^{-1}$, and (b) a mixture of 3:1 complexes in which the $(maltol)_2$ (glycine) iron(III) complex predominates, as a glass.

Determination of partition coefficients

The partition of coefficient $K_{part}$, being the ratio (concentration of compound in n-octanol)/(concentration of compound in aqueous phase) on partition between n-octanol and aqueous tris hydrochloride (20 mM, pH 7.4; tris represents 2-amino-2-hydroxymethylpropane 1,3-diol), is measured at 20°C for the 3:1 iron(III) complexes listed in the Table (at $10^{-4}$M) by spectophotometry, the complex prepared as described above being dissolved initially in the aqueous tris hydrochloride. Acid washed glassware is used throughout and, following mixing of 5 ml of the $10^{-4}$M aqueous solution with 5 ml of n-octanol for 1 minute, the aqueous n-octanol mixture is centrifuged at 1,000 g for 30 seconds. The two resulting phases are separated for a concentration determination by spectrophotometry on each, the range 340—640 nm being used. Values typical of those obtained are shown in the Table.

TABLE 1

Partition coefficients

| Iron(III) complex | Partition coefficient |
|---|---|
| $(maltol)_2$ (1-ethyl-3-hydroxypyrid-2-one) | 0.32 |
| (ethylpyromeconic acid)$_2$ (maltol) | 1.69 |
| (1-ethyl-3-hydroxypyrid-2-one)$_2$ (1-butyl-3-hydroxypyrid-4-one) | 3.56 |
| $(maltol)_2$ (ascorbic acid) | 0.14 |
| $(maltol)_2$ (leucine) | 0.17 |
| $(maltol)_2$ (glycine) | 0.18 |

[1] The concentration of the 1 molar equivalent ligand-providing compound is *ca.* 0.1M although this concentration may be varied, for example in a range of 0.1 to 1M, subject only to the solubility of the particular ligand-providing compounds in the solvent system being used.

Example 2

Donation of iron to apotransferrin

(Maltol)$_2$ (ascorbic acid) iron(III) was prepared *in situ* by dissolving maltol and ascorbic acid in a 2:1 molar ratio in an aqueous medium containing morpholine propane sulphonate (MOPS, 25 mM) and sodium hydrogen carbonate (30 mM), the pH being adjusted to 7.4 with hydrochloric acid (50% v/v). To the medium was added a solution of $^{59}$Fe enriched ferric chloride in 0.1M aqueous hydrochloric acid containing a molar amount of iron equivalent to that of the ascorbic acid. Equal volumes of the solution of the iron complex and of apotransferrin in the same aqueous medium were mixed to provide concentrations in the mixture of $5 \times 10^{-5}$M of apotransferrin and $1 \times 10^{-4}$M of iron.

The mixture was incubated at room temperature in separate experiments for 10 and 60 minutes and an aliquot was then added to a Sephadex G10 column equilibrated with the same aqueous medium described above and the column eluted with that medium, fractions being collected and counted. The percentages of $^{59}$Fe associated with apotransferrin and with ligands are calculated as cpm iron-apotransferrin/total cpm and cpm iron-(maltol)$_2$ (ascorbic acid)/total cpm.

It was found that after 10 minutes 43% of the $^{59}$Fe was associated with apotransferrin and 57% of the $^{59}$Fe with the ligands, whilst after 60 minutes the amount of $^{59}$Fe donated to the aptotransferrin had risen to 75%, only 25% remaining associated with the ligands.

Example 3

*In vitro* tests on permeation of iron complexes into human erythrocytes

The accumulation of iron by human erythrocytes which are associated with (ethylpyromeconic acid)$_2$ (maltol) iron(III), (maltol)$_2$ (etylpyromeconic acid) iron(III), (maltol)$_2$ (ascorbic acid) iron(III) and (1-ethyl-3-hydroxypyrid-2-one)$_2$ (1-butyl-3-hydroxypyrid-4-one) iron(III) was studied together with that of a group of four other iron compounds for comparative purposes, this group comprising the 3:1 homogeneous iron complexes, (ethylpyromeconic acid)$_3$ iron(III) and (maltol)$_3$ iron(III), and the salts, ferric NTA (nitrilotriacetic acid) and ferrous sulphate.

The iron compounds were used in solution in an aqueous medium containing tris (20 mM) and sodium chloride (130 mM), the pH being adjusted to 7.4 with hydrochloric acid (50% v/v). The solid ferric NTA and ferric sulphate, labelled with $^{59}$Fe, were dissolved in the aqueous medium to produce a $10^{-4}$M solution whilst the iron complexes were prepared *in situ* by solution of the ligand-providing compound, or of an appropriate molar ratio of ligand-providing compounds, in the aqueous medium and the addition of an appropriate molar proportion of $^{59}$Fe enriched ferric chloride in 0.1M aqueous hydrochloric acid, the final concentration of iron being $10^{-4}$M.

Packed human erythrocytes (0.5 ml) were incubated in the aqueous medium at 37°C in separate experiments for 5 and 30 minutes in all cases, and for the (maltol)$_2$ (ascorbic acid) iron(III) complex only, in a series of experiments conducted for 2, 5, 10, 15, 30, 45 and 60 minutes. Following incubation, an aliquot of the erythrocyte incubation medium mixture was separated by centrifugation over two leyers of silicone oil ($\rho = 1.07$, and $p = 1.2$ respectively). The $^{59}$Fe levels associated with each of the erythrocytes and the incubation medium were counted and are presented as a distribution ratio (concentration in erythrocytes/concentration in medium) in Table 2 for the 5 and 30 minute experiments and in Table 3 for the other experiment (maltol)$_2$ (ascorbic acid) iron(III) (all data in the Tables represents a mean of at least three independent experiments).

It will be seen from Table 2 that ethylpyromeconic acid is more effective as a ligand than maltol in producing uptake of an iron complex by erythrocytes, the uptake both for the complex containing three ethylpyromeconic acid ligands and that containing two of such ligands reaching an equilibrium after only 5 minutes. It should also be noted that higher level of iron uptake is achieved at 30 minutes for each of the three mixed ligand complexes containing a hydroxypyrone ligand than for either of the complexes containing three identical hydroxypyrone ligands.

TABLE 2

| Compound | Ligand concentration (mM) | Distribution ratio | |
|---|---|---|---|
| | | 5 minutes | 30 minutes |
| $Fe^{III}$(ethylpyromeconic acid)$_3$ | 0.3 | 3.7 | 3.6 |
| $Fe^{III}$(maltol)$_3$ | 0.3 | 0.2 | 1.1 |
| $Fe^{III}$(ethylpyromeconic acid)$_2$ (maltol) | 0.2:0.1 | 5.6 | 5.2 |
| $Fe^{III}$(maltol)$_2$(ethylpyromeconic acid) | 0.2:0.1 | 1.9 | 6.3 |
| $Fe^{III}$(maltol)$_2$(ascorbic acid) | 0.2:0.1 | 1.5 | 5.2 |
| $Fe^{III}$(1-ethyl-3-hydroxypyrid-2-one)$_2$(1-butyl-3-hydroxypyrid-4-one) | 0.2:0.1 | 0.4 | 1.4 |
| $Fe^{III}$ NTA | 0.1 | 6.2 | 4.9(1) |
| $Fe^{II}SO_4$ | 0.1 | 5.0 | 4.3(1) |

(1) An analysis of the erythrocyte membranes after incubation shows that these apparently high levels of iron uptake are due to binding to the cell membrane and do not in fact reflect true values of iron uptake.

TABLE 3

[$Fe^{III}$(maltol)$_2$(ascorbic acid)]

| Time (minutes) | Distribution ratio |
|---|---|
| 2 | 6.1 |
| 5 | 8.0 |
| 10 | 10.2 |
| 15 | 12.2 |
| 30 | 17.1 |
| 45 | 26.5(1) |
| 60 | 30.8(1) |

(1) These high values arise from dissociation of the complex and donation of iron to intracelluar protein.

Example 4

*In vitro* tests experimention of non-everted rat jejunal segments by iron complexes

The uptake of iron by non-everted rat jejunal segments was studied for (ethylpyromeonic acid)$_2$ (maltol) iron(III), (maltol)$_2$ (ethylpyromeconic acid) iron(III) and (1-ethyl-3-hydroxypyrid-2-one)$_2$ (1-butyl-3-hydroxypyrid-4-one) iron(III). For comparative purposes, similar experiments were carried out with (ethylpyromeconic acid)$_3$ iron(III) and (maltol)$_3$ iron(III), the latter complex being used alone and in admixture with an excess of maltol corresponding to either 1 molar proportion or 7 molar proportions are free maltol in addition to the 3 molar proportions contained in the complex in association with 1 molar proportion of iron(III).

The iron complexes were prepared *in situ* in oxygenated HEPES buffer by solution of the appropriate amount of the ligand-providing compound, or of an appropriate molar ratio of ligand-providing compounds, in the buffer and the addition of appropriate molar proportion of $^{59}$Fe enriched ferric chloride in 0.1M aqueous hydrochloric acid, the final concentration of iron being $10^{-4}$M.

Rats (male, Sprague Dawley, 70—90 g) were killed and the jejunum removed. It was divided into 3 cm lengths, each being cut lengthwise and opened out. These lengths were further cut into segments of 30—35 mg (3 per incubation flask). The jejunum was incubated at 37°C (pre-incubated flasks being used, each of which contained 3 segments) in a medium of oxygenated HEPES buffer containing the appropriate iron complex for 10 minutes. The tissue and medium were then counted.

The uptake of $^{49}$Fe was calculated as a distribution ratio (concentration in tisuse/concentration in medium) corrected for the water content (80%) and the extracellular fraction (found to be 20% by sulphate space studies) of the tissue. The results are shown in Table 4 and it will be seen, in contrast to the results obtained with erythrocytes, that the uptake of iron from the complex containing three ethylpyromeconic acid ligands is not greater than that for the complex containing three maltol ligands. However as in the erythrocyte experiments, the uptake of iron is greater for the three mixed ligand complexes containing a hydroxypyrone ligand than for either of the complexes containing three hydroxypyrone ligands.

TABLE 4

| Compound | Ligand concentration (mM) | Distribution ratio |
|---|---|---|
| $Fe^{(III)}(maltol)_3$ + maltol (1:7 molar ratio of complex: maltol) | 1 | 0.1 |
| $Fe^{(III)}(maltol)_3$ + maltol (1:1 molar ratio of complex: maltol) | 0.4 | 0.6 |
| $Fe^{(III)}(maltol)_3$ | 0.3 | 1.3 |
| $Fe^{(III)}$(ethylpyromeconic acid)$_3$ | 0.3 | 1.0 |
| $Fe^{(III)}$(ethylpyromeconic acid)$_2$(maltol) | 0.2:0.1 | 6.0 |
| $Fe^{(III)}(maltol)_2$(ethylpyromeconic acid) | 0.2:0.1 | 4.3 |
| $Fe^{(III)}(maltol)_2$(ascorbic acid) | 0.2:0.1 | 9.7 |
| $Fe^{(III)}$(1-ethyl-3-hydroxypyrid 2-one)$_2$-(1-butyl-3-hydroxy-3-methylpyrid-4-one) | 0.2:0.1 | 2.4 |

**Claims**

1. A neutral 3:1 ligand:iron(III) complex comprising three monobasic, bidentate ligands, of which at least two are different, in combination with a ferric cation, each ligand separately being provided by a compound which is:

(1) a 3-hydroxy-4-pyrone of formula (I)

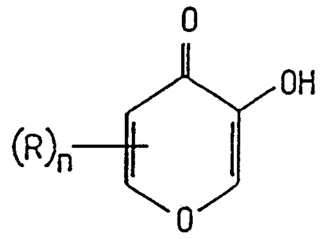

(I)

in which each R separately is an aliphatic or cycloaliphatic hydrocarbon group of 1 to 6 carbon atoms and n is 0, 1, 2 or 3;

(2) a 3-hydroxypyridone of formula (II) or (III)

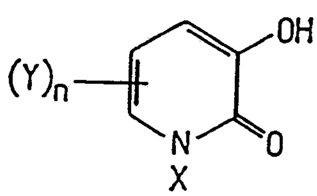

(II)

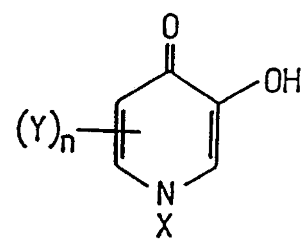

(III)

in which each Y separately is a $C_{1-6}$ aliphatic or cycloaliphatic hydrocarbon group or a $C_{1-3}$ alkyl group substituted by a $C_{1-4}$ alkoxy, halogen or hydroxy group.

X is a $C_{1-6}$ aliphatic or cycloaliphatic hydrocarbon group, a formyl or ($C_{1-4}$ alkyl)-carbonyl group, or a $C_{1-8}$ aliphatic or cycloaliphatic hydrocarbon group substituted by one or more substituents selected from formyl, ($C_{1-4}$)-carbonyl, $C_{1-4}$ alkoxy, carbamoyl, sulphamoyl, mono- and di- $C_{1-6}$ aliphatic or cycloaliphatic hydrocarbyl N-substituted carbamoyl and N-substituted sulphamoyl, formylamino, ($C_{1-6}$ aliphatic or cycloaliphatic hydrocarbyl)-carbonylamino, ($C_{1-6}$ aliphatic or cycloaliphatic hydrocarbyl)-sulphonylamino, mono-$C_{1-6}$ aliphatic or cycloaliphatic hydrocarbyl N-substituted formylamino, N-substituted ($C_{1-6}$ aliphatic or cycloaliphatic hydrocarbyl)-carbonylamino and N-substituted ($C_{1-6}$ aliphatic or cycloaliphatic hydrocarbyl)-sulphonylamino, formyloxy, ($C_{1-6}$ aliphatic or cycloaliphatic hydrocarbyl)-carbonyloxy, ($C_{1-6}$ aliphatic or cycloaliphatic hydrocarbyl)-oxycarbonyl, ($C_{1-6}$ aliphatic or cycloaliphatic hydrocarbyl)-sulphonyloxy, ($C_{1-6}$ aliphatic or cycloaliphatic hydrocarbyl)-oxysulphonyl, halogen and hydroxy groups, and n is 0, 1, 2 or 3, but excluding compounds in which the grouping

NX contains either a group N—C—N or a group N—C—O—; or

(3) lactic acid, gluconic acid, ascorbic acid, glycine, leucine, isoleucine, methionine, phenylalanine, tyrosine, valine or a di- or tri-peptide in which the amino acid residues, which may be the same or different, are residues of amino acids selected from this last mentioned group or seven amino acids; but with the proviso that at least one of the ligands is of type (1) or (2).

2. A complex according to Claim 1, in which at least two of the ligands are provided by compounds of different types among (1), (2) and (3).

3. A complex according to Claim 1 or 2, in which two of the ligands are provided by the same compound and the other ligand is provided by a different compound.

4. A complex according to Claim 1, 2 or 3, in which the compound of type (3) is ascorbic acid.

5. A complex according to any of Claims 1 to 4, in which at least one ligand is provided by a compound of type (1).

6. A complex according to Claim 1 or 3, in which each ligand separately is provided by a compound of type (1).

7. A complex according to any of the preceding claims, in which the compound of type (1) is a 3-hydroxy-4-pyrone of formula (I) wherein n is 0, or n is 1, 2 or 3 and each R separately is a methyl, ethyl, n-propyl or isopropyl group.

8. A complex according to any of the preceding claims, in which the compound of type (1) is a 3-hydroxy-4-pyrone of formula (I) wherein n is 0, or n is 1 and the single group R is present at the 2- or 6-position.

9. A complex according to Claim 7, in which the compound of type (1) is selected from the group consisting of 3-hydroxy-4-pyrone, 3-hydroxy-2-methyl-4-pyrone, 3-hydroxy-6-methyl-4-pyrone and 2-ethyl-3-hydroxy-4-pyrone.

10. A complex according to Claim 7, in which the compound of type (1) is selected from the group consisting of 3-hydroxy-2-methyl-4-pyrone and 2-ethyl-3-hydroxy-4-pyrone.

11. A complex according to any of Claims 1 to 5, in which at least one ligand is provided by a compound of type (2).

12. A complex according to Claim 1 or 3, in which each ligand separately is provided by a compound of type (2).

13. A complex according to any of Claims 1 to 4, 11 and 12, in which the compound of type (2) is a 3-hydroxypyridone of formula (II) or (III) in which X is an aliphatic or cycloaliphatic hydrocarbon group of 1 to 6 carbon atoms and each Y separately is an aliphatic or cycloaliphatic hydrocarbon group of 1 to 6 carbon atoms.

14. A complex according to any of Claims 1 to 4, 11, 12 and 13, in which the compound of type (2) is of formula (II).

15. A complex according to Claim 13, in which the compound of type (2) is selected from the group consisting of 3-hydroxy-1-methylpyrid-2-one, 1-ethyl-3-hydroxypyrid-2-one, 3-hydroxy-1-propylpyrid-2-one, 3-hydroxy-1-(1'-methylethyl)-pyrid-2-one, 3-hydroxy-1,2-dimethylpyrid-4-one, 1-ethyl-3-hydroxy-2-methylpyrid-4-one, 3-hydroxy-2-methyl-1-propylpyrid-4-one and 3-hydroxy-1-(1'-methylethyl)-2-methylpyrid-4-one.

16. A complex according to Claim 1, being (1-ethyl-3-hydroxypyrid-2-one)$_2$ (1-butyl-3-hydroxypyrid-4-one) iron(III), (3-hydroxy-2-methyl-4-pyrone)$_2$ (1-ethyl-3-hydroxypyrid-2-one) iron(III), (3-hydroxy-2-methyl-4-pyrone)$_2$ (leucine) iron(III), (3-hydroxy-2-methyl-4-pyrone)$_2$ (glycine) iron(III), (3-hydroxy-2-methyl-4-pyrone)$_2$ (ascorbic acid) iron(III), (3-hydroxy-2-methyl-4-pyrone)$_2$ (gluconic acid) iron(III), (3-hydroxy-2-methyl-4-pyrone)$_2$ (2-ethyl-3-hydroxy-4-pyrone) iron(III) or (2-ethyl-3-hydroxy-4-pyrone)$_2$ (3-hydroxy-2-methyl-4-pyrone) iron(III).

17. A complex according to Claim 1, being (3-hydroxy-2-methyl-4-pyrone)$_2$ (2-ethyl-3-hydroxy-4-pyrone) iron(III) or (2-ethyl-3-hydroxy-4-pyrone)$_2$ (3-hydroxy-2-methyl-4-pyrone) iron(III).

18. A complex according to any of the preceding claims, in which the neutral 3:1 complex is substantially free from any 1:1 or 2:1 iron(III) complex in which the ligands are selected from those present in the 3:1 complex.

19. A pharmaceutical composition comprising an iron complex according to any of Claims 1 to 18, together with a physiologically acceptable diluent or carrier.

20. A pharmaceutical composition according to Claim 19 in solid form.

21. A pharmaceutical composition according to Claim 19 or 20, which is adapted for oral or buccal administration.

22. A pharmaceutical composition according to Claim 19, 20 or 21, in which the complex or complexes are encapsulated by a material resistant to dissociation under aqueous acidic conditions.

23. A pharmaceutical composition according to Claim 19 in aerosol form.

24. A pharmaceutical composition according to Claim 19, which contains a sterile, pyrogen-free diluent.

25. A animal foodstuff comprising an iron complex according to any Claims 1 to 18.

26. A process for the preparation of an iron complex according to any of Claims 1 to 18 which comprises adding the ligand-providing compounds to ferric ions and effecting reaction therewith, and thereafter treating the reaction mixture to effect isolation of the iron complex.

27. An iron complex according to any of Claims 1 to 18 for use in therapy.

28. The use of an iron complex according to any of Claims 1 to 18 for the manufacture of a medicament for use in effecting an increase in the levels of iron in a patient's bloodstream.

**Patentansprüche**

1. Neutraler 3:1 Ligand:Eisen (III) Komplex, dadurch gekennzeichnet, daß er drei einbasische, zweizähnige Liganden, von denen mindestens zwei verschieden sind, in Kombination mit einem Eisen (III)-kation enthält, wobei jeder Ligand separat durch eine Verbindung zur Verfügung gestellt wird, die:

(1) ein 3-Hydroxy-4-pyron der Formel (I)

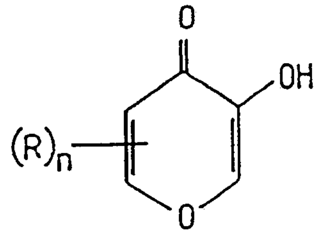

(I)

worin jedes R separat eine aliphatische oder cycloaliphatische Kohlenwasserstoffgruppe mit 1 bis 6 Kolenstoffatomen ist und n für 0, 1, 2 oder 3 steht:

(2) ein 3-Hydroxypyridon der Formel (II) oder (III)

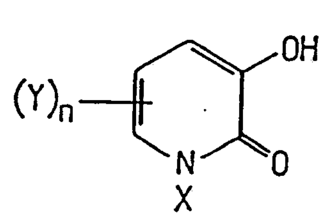

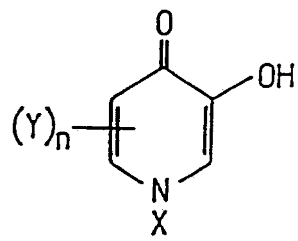

(II) (III)

worin jedes Y separat ein $C_{1-6}$ aliphatische oder cycloaliphatische Kohlenwasserstoffgruppe oder eine $C_{1-3}$ Alkylgruppe, substituiert durch eine $C_{1-4}$ Alkoxy-, Halogen- oder Hydroxygruppe, ist, X eine $C_{1-6}$ aliphatische oder cycloaliphatische Kohlenwasserstoffgruppe, eine Formyl- oder ($C_{1-4}$ Alkyl)-carbonylgruppe oder eine $C_{1-8}$ aliphatische oder cycloaliphatische Kohlenwasserstoffgruppe, substituiert durch einen oder mehrere Substituenten, ausgewählt aus Formyl-, ($C_{1-4}$ Alkyl)-carbonyl-, $C_{1-4}$ Alkoxy, Carbamoyl-, Sulphamoyl-, mono- und di- $C_{1-6}$ aliphatische oder cycloaliphatische Hydrocarbyl-, N-substitutierte Carbamoyl- und N-substituierte Sulphamoyl-, Formylamino-, ($C_{1-6}$ aliphatische oder cycloaliphatische Hydrocarbyl)-carbonylamino-, ($C_{1-6}$ aliphatische oder cycloaliphatische Hydrocarbyl)-sulphonylamino-, mono-$C_{1-6}$ aliphatische oder cycloaliphatische Hydrocarbyl N-substituierte Formylamino-, N-substituierte ($C_{1-6}$ aliphatische oder cycloaliphatische Hydrocarbyl)-carbonylamino- und N-substituierte ($C_{1-6}$ aliphatische oder cycloaliphatische Hydrocarbyl)-sulphonylamino-, Formyloxy-, $C_{1-6}$ aliphatische oder cycloaliphatische Hydrocarbyl)-carbonyloxy-, ($C_{1-6}$ aliphatische oder cycloaliphatische Hydrocarbyl)-oxycarbonyl-, ($C_{1-6}$ aliphatische oder cycloaliphatische Hydrocarbyl)-sulphonyloxy-, ($C_{1-6}$ aliphatische oder cycloaliphatische Hydrocarbyl)-oxysulphonyl-, Halogen und Hydroxygruppen ist, und n für 0, 1, 2 oder 3 steht, aber mit Ausschluß der Verbindugen, in denen die Gruppierung NX entweder eine Gruppe

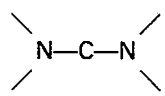 oder eine Gruppe 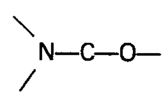

enthält; oder

(3) Milchsäure, Gluconsäure, Ascorbinsäure, Glycin, Leucin, Isoleucin, Methionin, Phenylalanin, Tyrosin, Valin oder ein di- oder tri-Peptid, worin die Aminosäurereste, die gleich oder verschieden sein können, Reste von Aminosäuren, ausgewählt aus der zuletzt erwähnten Gruppe von sieben Aminosäuren sind, ist aber mit der Maßgabe, daß mindestens einer der Liganden vom Typ (1) oder (2) ist.

2. Komplex nach Anspruch 1, dadurch gekennzeichnet, daß mindestens zwei der Liganden durch Verbindungen von verschiedenen Typen unter (1), (2) und (3) zur Verfügung gestellt werden.

3. Komplex nach Anspruch 1 oder 2, dadurch gekennzeichnet, daß zwei der Liganden durch die gleiche Verbindung und der weitere Ligand durch eine verschiedene Verbindung zur Verfügung gestellt werden.

4. Komplex nach Anspruch 1, 2 oder 3, dadurch gekennzeichnet, daß die Verbindung vom Typ (3) Ascorbinsäure ist.

5. Komplex nach einem der Ansprüche 1 bis 4, dadurch gekennzeichnet, daß mindestens ein Ligand durch eine Verbindung vom Typ (1) zur Verfügung gestellt wird.

6. Komplex nach einem der Anspruche 1 oder 3, dadurch gekennzeichnet, daß jeder Ligand separat durch eine Verbindung vom Typ (1) zur Verfügung gestellt wird.

7. Komplex nach einem der vorausgehenden Ansprüche, dadurch gekennzeichnet, daß die Verbindung vom Typ (1) ein 3-Hydroxy-4-pyron der Formel (I) ist, worin n für 0 steht oder n für 1, 2 oder 3 steht und

jedes R separat eine Methyl-, Ethyl-, n-Propyl- oder Isopropylgruppe bedeutet.

8. Komplex nach einem der vorausgehenden Ansprüche, dadurch gekennzeichnet, daß die Verbindung vom Typ (1) ein 3-Hydroxy-4-pyron der Formel (I) ist, worin für 0 steht, oder n für 1 steht und die einzelne Gruppe R in der 2- oder 6-Position anwesend ist.

9. Komplex nach Anspruch 7, dadurch gekennzeichnet, daß die Verbindung vom Typ (1) aus der Gruppe, bestehend aus 3-Hydroxy-4-pyron, 3-Hydroxy-2-methyl-4-pyron, 3-Hydroxy-6-methyl-4-pyron und 2-Ethyl-3-hydroxy-4-pyron ausgewählt ist.

10. Komplex nach Anspruch 7, dadurch gekennzeichnet, daß die Verbindung vom Typ (1) aus der Gruppe, bestehend aus 3-Hydroxy-2-methyl-4-pyron und 2-Ethyl-3-hydroxy-4-pyron ausgewählt ist.

11. Komplex nach einem der Ansprüche 1 bis 5, dadurch gekennzeichnet, daß mindestens ein Ligand durch eine Verbindung vom Typ (2) zur Verfügung gestellt wird.

12. Komplex nach einem der Anspruch 1 oder 3, dadurch gekennzeichnet, daß jeder Ligand separat durch eine Verbindung von Typ (2) zur Verfügung gestellt wird.

13. Komplex nach einem der Ansprüche 1 bis 4, 11 und 12, dadurch gekennzeichnet, daß die Verbindung von Typ (2) ein 3-Hydroxypyridon der Formel (II) oder (III) ist, worin X für eine aliphatische oder cycloaliphatische Kohlenwasserstoffgruppe mit 1 bis 6 Kohlenstoffatomen und jedes Y separat für eine aliphatische oder cycloaliphatische Kohlenwasserstoffgruppe mit 1 bis 6 Kohlenstoffatomen steht.

14. Komplex nach einem der Ansprüche 1 bis 4, 11, 12 und 13, dadurch gekennzeichnet, daß die Verbindung vom Typ (2) die Formel (II) besitzt.

15. Komplex nach Anspruch 13, dadurch gekennzeichnet, daß die Verbindung von Typ (2) aus der Gruppe, bestehend aus 3-Hydroxy-1-methylpyrid-2-on, 1-Ethyl-3-hydroxypyrid-2-on, 3-Hydroxy-1-propylpyrid-2-on, 3-Hydroxy-1-(1′-methylethyl)-pyrid-2-on, 3-Hydroxy-1,2-dimethylpyrid-4-on, 1-Ethyl-3-hydroxy-2-methylpyrid-4-on, 3-Hydroxy-2-methyl-1-propylpyrid-4-on und 3-Hydroxy-1-(1′-methylethyl)-2-methylpyrid-4-on ausgewählt ist.

16. Komplex nach Anspruch 1, dadurch gekennzeichnet, daß er (1-Ethyl-3-hydroxypyrid-2-on)$_2$ (1-butyl-3-hydroxypyrid-4-on)-Eisen(III), (3-Hydroxy-2-methyl-4-pyron)$_2$ (1-ethyl-3-hydroxypyrid-2-on)-Eisen(III), (3-Hydroxy-2-methyl-4-pyron)$_2$ (leucin)-Eisen(III), (3-Hydroxy-2-methyl-4-pyron)$_2$ (glycin)-Eisen(III), (3-Hydroxy-2-methyl-4-pyron)$_2$ (Ascorbinsäure)-Eisen(III), (3-Hydroxy-2-methyl-4-pyron)$_2$ (Gluconsäure)-Eisen(III), (3-Hydroxy-2-methyl-4-pyron)$_2$ (2-ethyl-3-hydroxy-4-pyron)-Eisen(III) oder (2-Ethyl-3-hydroxy-4-pyron)$_2$ (3-hydroxy-2-methyl-4-pyron)-Eisen(III) ist.

17. Komplex nach Anspruch 17, dadurch gekennzeichnet, daß er (3-Hydroxy-2-methyl-4-pyron)$_2$ (2-ethyl-3-hydroxy-4-pyron)-Eisen(III) oder (2-Ethyl-3-hydroxy-4-pyron)$_2$ (3-hydroxy-2-methyl-4-pyron)-Eisen(III) ist.

18. Komplex nach einem der vorausgehenden Ansprüche, dadurch gekennzeichnet, daß der neutrale 3:1 Komplex im wesentlichen frei von irgendeinem 1:1 oder 2:1 Eisen(III) Komplex ist, worin die Liganden aus denen, die in dem 3:1 Komplex anwesend sind, ausgewählt sind.

19. Pharmazeutisches Präparat, dadurch gekennzeichnet, daß es einen Eisenkomplex nach einem der Ansprüche 1 bis 18, zusammen mit einem physiologisch annehmbaren Diluenten oder Träger enthält.

20. Pharmazeutisches Präparat nach Anspruch 19, dadurch gekennzeichnet, daß es in fester Form vorliegt.

21. Pharmazeutisches Präparat nach Anspruch 19 oder 20, dadurch gekennzeichnet, daß es der oralen oder bukkalen Verabreichung angepaßt ist.

22. Pharmazeutisches Präparat nach Anspruch 19, 20 oder 21, dadurch gekennzeichnet, daß der bzw. die Komplex Komplexe durch ein Material, das gegenüber Dissoziation unter wäßrigen sauren Bedingungen beständig ist, eingekapselt ist bzw. sind.

23. Pharmazeutisches Präparat nach Anspruch 19, dadurch gekennzeichnet, daß es in Aerosolform vorliegt.

24. Pharmazeutisches Präparat nach Anspruch 19, dadurch gekennzeichnet, daß einen sterilen, pyrogenfreien Diluenten enthält.

25. Tierfuttermittel, dadurch gekennzeichnet, daß es einen Eisenkomplex nach einem der Ansprüche 1 bis 18 umfaßt.

26. Verfahren zur Herstellung eines Einsekomplexes nach einem der Ansprüche 1 bis 18, dadurch gekennzeichnet, daß man die Liganden-zur-Verfügung-stellenden Verbindung den Eisen(III)-Ionen hinzufügt une eine Reaktion damit bewirkt und daß man anschließend das Reaktionsgemisch behandelt, um den Eisenkomplex zu isolieren.

27. Eisenkomplex nach einem der Ansprüche 1 bis 18 zur therapeutischen Verwendung.

28. Verwendung eines Eisenkomplexes nach einem der Ansprüche 1 bis 18 zur Herstellung eines Medikaments zur Verwendung zur Bewirkung einer Erhöhung des Bluteisenspiegels eines Patienten.

**Revendications**

1. Complexe neutre à 3:1 ligand:fer(III), comprenant trois ligands bidentés monobasiques, dont deux au moins sont différents, en combinaison avec un cation ferrique, chaque ligand étant fourni séparément par un composé qui est:

(1) une 3-hydroxy-4-pyrone de formule (I)

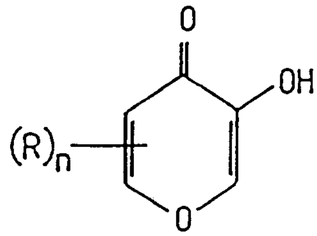

(I)

dans laquelle chaque symbole R représente, séparément, un groupe hydrocarboné aliphatique ou cycloaliphatique ayant 1 à 6 atomes de carbone, et n vaut 0, 1, 2 ou 3;

(2) une 3-hydroxypyridone de formule (II) ou (III)

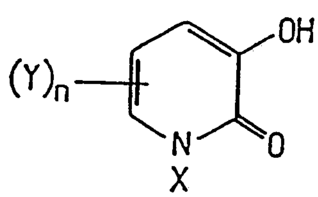

(II)

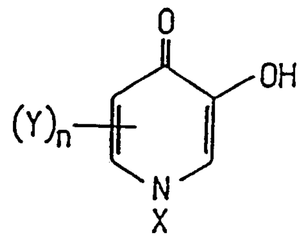

(III)

dans lesquelles chaque symbole Y représente, séparément, un groupe hydrocarboné aliphatique ou cycloaliphatique en $C_1$ à $C_6$ ou un groupe alkyle en $C_1$ à $C_3$ substitué par an groupe alcoxy en $C_1$ à $C_4$, halogéno ou hydroxyle,

X représente un groupe hydrocarboné alipathique ou cycloaliphatique comportant 1 à 6 atomes de carbone, un groupe formyle ou un groupe alkyl (en $C_1$ à $C_4$) carbonyle, ou un groupe hydrocarboné aliphatique ou cycloaliphatique en $C_1$ à $C_8$ substitué par un ou plusieurs substituants choisis parmi un reste formyle, alkyle, alkyl (en $C_1$ à $C_4$)-carbonyle, alcoxy en $C_1$ à $C_4$, carbamoyle, sulfamoyle, carbamoyle et sulfamoyle (monosubstitués ou disubstitués sur l'azote (par un ou deux groupes hydrocarbyles aliphatiques ou cycloaliphatiques en $C_1$ à $C_6$), un reste formylamino, hydrocarbyl (aliphatique ou cycloaliphatique en $C_1$ à $C_6$)-carbonylamino, hydrocarbyl (aliphatique ou cycloaliphatique en $C_1$ à $C_6$) sulfonylamino, formylamino (monosubstitué sur l'azote par un reste hydrocarbyle aliphatique ou cycloaliphatique en $C_1$ à $C_6$, carbonylamino (substitué sur l'azote par un reste hydrocarbyle aliphatique ou cycloaliphatique en $C_1$ à $C_6$) et sulfonylamino (substitué sur l'azote par un reste hydrocarbyle aliphatique ou cycloaliphatique en $C_1$ à $C_6$) formyloxy, hydrocarbyl (aliphatique ou cycloaliphatique en $C_1$ à $C_6$)-carbonyloxy, hydrocarbyl (aliphatique ou cycloaliphatique en $C_1$ à $C_6$)-oxycarbonyle, hydrocarbyl (aliphatique ou cycloaliphatique en $C_1$ à $C_6$)-sulfonyloxy, hydrocarbyl (aliphatique ou cycloaliphatique en $C_1$ à $C_6$) oxysulfonyle, halogéno et hydroxy, et n vaut 0, 1, 2 ou 3, mais à l'exclusion de composés dans lesquels le groupement

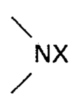 contient un groupe 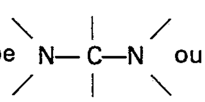 ou un groupe 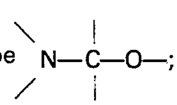 ou

(3) l'acide lactique, l'acide gluconqiue, l'acide ascorbique, la glycine, la leucine, l'isoleucine, la méthionine, la phénylalanine, la tyrosine, la valine ou un di-peptide ou tri-peptide dans lesquels les restes aminoacides, qui peuvent être identiques ou différents, sont des restes d'aminoacides choisis parmi le groupe mentionée en dernier lieu comportant sept aminoacides; mais à la condition qu'au moins l'un des ligands appartienne au type (1) ou (2).

2. Complexe selon la revendication 1, dans lequel au moins deux des ligands sont fournis par des composés de types différents choisis parmi (1) (2) et (3).

3. Complexe selon la revendication 1 ou 2, dans lequel deux des ligands sont fournis par le même composé et l'autre ligand est fourni par un composé différent.

4. Complexe selon la revendication 1, 2, ou 3, dans lequel le composé de type (3) est l'acide ascorbique.

5. Complexe selon l'une quelconque des revendications 1 à 4, dans lequel au moins un ligand est fourni par un composé de type (1).

6. Complexe selon la revendication 1 ou 3, dans lequel chaque ligand est fourni séparément par un composé de type (1).

7. Complexe selon l'une quelconque des revendications précédentes, dans lequel le composé de type (1) est une 3-hydroxy-4-pyrone de formule (I), dans laquelle n est nul ou bien n vaut 1, 2 ou 3, est chaque R représente séparément un groupe méthyle, éthyle, n-propyle ou isopropyle.

8. Complexe selon l'une quelconque des revendications précédentes, dans lequel le composé de type (1) est une 3-hydroxy-4-pyrone de formule (I), dans laquelle n est nul n vaut 1, et le groupe R unique est présente en position 2 ou en position 6.

9. Complexe selon la revendication 7, dans lequel le composé du type (1) est choisi dans l'ensemble constitué par la 3-hydroxy-4-pyrone, la 3-hydroxy-2-méthyl-4-pyrone, la 3-hydroxy-6-méthyl-4-pyrone et la 2-éthyl-3-hydroxy-4-pyrone.

10. Complexe selon la revendication 7, dans lequel le composé de type (1) est choisi dans l'ensemble constitué par la 3-hydroxy-2-méthyl-4-pyrone et la 2-éthyl-3-hydroxy-4-pyrone.

11. Complexe selon l'une quelconque des revendications 1 à 5, dans lequel ou moins un ligand est fourni par un composé de type (2).

12. Complexe selon la revendication 1 ou 3, dans lequel chaque ligand est séparément fourni par un composé de type (2).

13. Complexe selon l'une quelconque des revendications 1 à 4, 11 et 12, dans lequel le composé de type (2) est une 3-hydroxypyridone de formule (II) ou (III), dans laquelle X représente un groupe hydrocarboné aliphatique ou cycloaliphatique ayant 1 à 6 atomes de carbone, et chaque Y représente, séparément, un groupe hydrocarboné aliphatique ou cycloaliphatique ayant 1 à 6 atomes de carbone.

14. Complexe selon l'une quelconque des revendications 1 à 4, 11, 12 et 13, dans lequel le composé de type (2) répond à la formule (II).

15. Complexe selon la revendication 13, dans lequel le composé de type (2) est choisi dans l'ensemble constitué par la 3-hydroxy-1-méthylpyrid-2-one, la 1-éthyl-3-hydroxypyrid-2-one, la 3-hydroxy-1-propylpyrid-2-one, la 3-hydroxy-1-(1'-méthyléthyl)-pyrid-2-one, la 3-hydroxy-1,2-diméthylpyrid-4-one, la 1-éthyl-3-hydroxy-2-méthylpyrid-4-one, la 3-hydroxy-2-méthyl-1-propylpyrid-4-one et le 3-hydroxy-1-(1'-méthyléthyl)-2-méthylpyrid-4-one.

16. Complexe selon la revendication 1, qui est le (1-éthyl-3-hydroxypyrid-2-one)$_2$ (1-butyl-3-hydroxypyrid-4-one) fer(III), le (3-hydroxy-2-méthyl-4-pyrone)$_2$ (1-éthyl-3-hydroxypyrid-2-one) fer(III), le (3-hydroxy-2-méthyl-4-pyrone)$_2$ (leucine) fer(III), le (3-hydroxy-2-méthyl-4-pyrone)$_2$ (glycine) fer(III), le (3-hydroxy-2-méthyl-4-pyrone)$_2$ (acide ascorbique) fer(III), le (3-hydroxy-2-méthyl-4-pyrone)$_2$ (acid gluconqiue) fer(III), le (3-hydroxy-2-méthyl-4-pyrone)$_2$ (2-éthyl-3-hydroxy-4-pyrone) fer(III), ou le (2-éthyl-3-hydroxy-4-pyrone)$_2$ (3-hydroxy-2-méthyl-4-pyrone) fer(III).

17. Complexe selon la revendication 1, qui est le (3-hydroxy-2-méthyl-pyrone)$_2$ (2-éthyl-3-hydroxy-4-pyrone) fer(III) ou le (2-éthyl-3-hydroxy-4-pyrone)$_2$ (3-hydroxy-2-méthyl-4-pyrone) fer(III).

18. Complexe selon l'une quelconque des revendications précédentes, dans lequel le complex neutre à 3:1 et sensiblement dépourvu de tout complexe à 1:1 ou à 2:1 de fer(III), dans lequel les ligands sont choisis parmi ceux présents dans le complex à 3:1.

19. Composition pharmaceutique comprenant un complexe der fer selon l'une quelconque des revendications 1 à 18 avec un diluant, excipient, véhicule ou support physiologiquement acceptable.

20. Composition pharmaceutique selon la revendication 19, sous forme solide.

21. Composition pharmaceutique selon la revendication 19 ou 20, qui est adaptée à une administration par voie orale ou buccale.

22. Composition pharmaceutique selon la revendication 19, 20, ou 21, dans laquelle le ou les complexes est ou sont encapsulés par une matière résistant à une dissociation dans des conditions de milieu aqueux acide.

23. Composition pharmaceutique selon la revendication 19, sous forme d'aérosol.

24. Composition pharmaceutique selon la revendication 19, qui contient un diluant stérile, dépourvu de pyrogènes.

25. Un aliment pour des animaux, cet aliment comprenant un complexe de fer selon l'une quelconque des revendications 1 à 18.

26. Procédé pour préparer un complexe de fer selon l'une quelconque des revendications 1 à 18, qui comprend l'addition des composés fournisseurs de ligands à des ions ferriques et la réalisation d'une réaction avec ces ions, puis le traitement du mélange réactionnel pour réaliser l'isolement du complexe de fer.

27. Complexe de fer selon l'une quelconque des revendications 1 à 18, destiné à servir en thérapie.

28. Utilisation d'un complexe de fer selon l'une quelconque des revendications 1 à 18 pour la fabrication d'un médicament destiné à servir à réaliser une augmentation des taux de fer dans un courant sanguin d'un patient.